# EUROPÄISCHE PATENTSCHRIFT

(11) **EP 2 760 513 B1**
(45) Veröffentlichungstag und Bekanntmachung des Hinweises auf die Patenterteilung: **27.07.2016**
(21) Anmeldenummer: 12770076.3
(22) Anmeldetag: 27.09.2012
(51) Int. Cl.: A61M 16/16, A61M 16/10, A61M 16/08

(54) **BEATMUNGSSCHLAUCHSYSTEM**
BREATHING TUBE SYSTEM
SYSTÈME DE TUBES DE RESPIRATION

(30) Priorität: 01.10.2011 DE 102011054131
(43) Veröffentlichungstag der Anmeldung: 06.08.2014
(73) Patentinhaber: Hamilton Bonaduz AG, 7402 Bonaduz (CH)
(72) Erfinder: BÜCHI, Rudolf, CH-7000 Chur (CH); FREI, Reto, CH-7402 Bonaduz (CH); MAEDER, Marc, CH-7208 Malans (CH); GRANZOTTO, Thomas, CH-7206 Igis (CH); ZOLKOS, Axel, 8864 Reichenburg (CH)
(74) Vertreter: Caspary, Karsten
(86) Internationale Anmeldenummer: PCT/EP2012/069107
(87) Internationale Veröffentlichungsnummer: WO 2013/045563

(56) Entgegenhaltungen:
- EP-A2- 0 672 430
- EP-A2- 2 229 973
- WO-A1-03/047671
- DE-B3-102008 039 137
- US-A1- 2006 283 447
- US-A1- 2011 108 031

## Beschreibung

Die vorliegende Erfindung betrifft ein Beatmungsschlauchsystem zum Beatmen von Patienten mit Atemgas sowie ein Beatmungssystem umfassend ein Beatmungsgerät, einen Atemluftbefeuchter und ein Beatmungsschlauchsystem.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen in üblicher Weise mit destilliertem Wasser gefüllten Flüssigkeitsbehälter auf, durch den das Einatemgas geleitet und mit Feuchtigkeit angereichert wird.

Um eine Kondensation von Feuchtigkeit innerhalb des Beatmungsschlauchsystems zu verhindern, sind der Inspirationsschlauch und der Exspirations- oder Ausatemschlauch normalerweise mit einer elektrischen Schlauchheizung versehen, die in Betrieb das durchströmende Ein- oder Ausatemgas erwärmt. Dabei wird beispielsweise eine sich integral innerhalb des Inspirations- oder Exspirationsschlauchs erstreckende Heizdrahtschleife verwendet oder der Inspirations- oder Exspirationsschlauch ist jeweils mit einer Heizdrahtwendel umwickelt.

Die Regelung der Atemgastemperatur erfolgt im Allgemeinen mit Hilfe eines nahe am Patienten angeordneten Temperatursensors, der mittels einer elektrischen Messleitung mit einer Steuereinrichtung, die beispielsweise im Atemluftbefeuchter angeordnet ist, verbunden ist.

Derartige Beatmungsschlauchsysteme sind zum Beispiel aus der DE 10 2008 039 137 B3, der DE 10 2007 003 455 A1, der DE 44 41 380 A1 oder der EP 1 338 297 A1 bekannt. Nachteilig an den Beatmungsschlauchsystemen des Standes der Technik ist, dass neben den pneumatischen Verbindungen von Inspirations- und Exspirationsschlauch mit Beatmungsgerät, Atemluftbefeuchter und Y-Stück am Patienten auch die elektrischen Leitungen für die Schlauchheizung und den Temperatursensor als eigenständige, lose Kabel vorhanden sind und unabhängig von einander angeschlossen werden müssen. Die Vielzahl an zu verbindenden Kabeln und Schläuchen führt zu Zeitverlust und möglicher Verwirrung beim Bedienpersonal, ist störend in der Patientenumgebung und anfällig für Beschädigungen. Weiterhin birgt die Verwendung von solchen losen Kabeln die Gefahr, dass sich die Bedienpersonen oder der Patient darin verheddern und die elektrische Verbindung zwischen dem Atemluftbefeuchter und den Heizdrähten schlimmstenfalls während der Behandlung des Patienten abreißt.

In der EP 2 229 973 A2 ist ein Beatmungsschlauchsystem beschrieben, das einen beheizbaren Inspirationsschlauch und zwei Exspirationsschläuche aufweist: Der Inspirationsschlauch ist an einem Ende mit einem Y-Verbinder und an dem anderen Ende mit einem T-Verbinder verbunden, wobei zwischen dem T-Verbinder und dem Y-Verbinder ein Atemluftbefeuchter angeordnet ist, durch den die Luft strömt und zusätzlich angefeuchtet wird, bevor sie zu einem Patienten gelangt. Eine separate Wärmeregulierungseinheit reguliert die Temperatur der Schläuche, 30, die über einzeln geführte Stromversorgungsleitungen jeweils an entsprechenden Muffen an die Schläuche angesteckt sind.

Es ist daher die Aufgabe der vorliegenden Erfindung, ein Beatmungsschlauchsystem bereit zu stellen, das die Anzahl der Schläuche und elektrischen Leitungen sowie deren Anschlüsse minimiert, flexible Anschlussmöglichkeiten bereit stellt und eine Verbindung von pneumatischen und elektrischen Anschlüssen in einem Verbindungsvorgang ermöglicht.

Diese Aufgabe wird durch die Merkmale des Anspruchs 1 gelöst. Vorteilhafte Ausgestaltungen und Ausführungsformen sind Gegenstand der Unteransprüche.

Erfindungsgemäß wird ein Beatmungsschlauchsystem für Atemgas zum Beatmen von Patienten bereit gestellt mit einem ersten Inspirationsschlauch, der mit einem Atemluftbefeuchter und mit einem Y-Stück verbindbar ist, einem zweiten Inspirationsschlauch, der mit dem Beatmungsgerät und dem Atemluftbefeuchter verbindbar ist, und einem Exspirationsschlauch, der mit dem Beatmungsgerät und dem Y-Stück verbindbar ist, wobei der erste Inspirationsschlauch und der Exspirationsschlauch wenigstens abschnittsweise jeweils eine Schlauchheizung mit mindestens einem Heizdraht aufweisen, die von einer Stromversorgungs- und Steuereinrichtung innerhalb des Atemluftbefeuchters steuerbar ist. Das Beatmungsschlauchsystem zeichnet sich dadurch aus, dass eine Stromversorgungsleitung der Schlauchheizung des Exspirationsschlauches ausgehend vom Anschluss des zweiten Inspirationsschlauches am Atemluftbefeuchter über den zweiten Inspirationsschlauch und ein vom Beatmungsgerät separates Verbindungselement geführt ist. Durch die weitgehende Integration der elektrischen Leitungen in die pneumatischen Schlauchverbindungen verbessert sich die Ergonomie für den Benutzer erheblich, denn es wird die Möglichkeit für Fehlbedienungen und technische Defekte reduziert. Darüber hinaus liegen weniger Schläuche bzw. Kabel frei, wodurch das Gesamtsystem weniger Platz verbraucht und weniger störanfällig ist.

Bevorzugt weisen der zweite Inspirationsschlauch und der erste Inspirationsschlauch jeweils ein Anschlussstück auf, das mit dem Atemluftbefeuchter derart verbindbar ist, dass die pneumatische Verbindung der Atemgaswege zusammen, d. h. im Wesentlichen gleichzeitig bzw. in einem Verbindungsvorgang, mit der elektrischen Verbindung bewirkt wird. Dies reduziert die Fehleranfälligkeit und erhöht die Benutzerfreundlichkeit des Gesamtsystems noch weiter, denn die pneumatischen Verbindungen müssen nicht mehr separat von den elektrischen Verbindungen hergestellt werden.

Vorteilhafter Weise ist das Verbindungselement als flexible Brücke ausgebildet und verbindet den Exspirationsschlauch und den zweiten Inspirationsschlauch im Bereich den beatmungsgeräteseitigen Enden, d. h. den Enden, die an das Beatmungsgerät anschließbar sind. Dadurch kann vermieden werden, dass das Verbindungselement eine potentiell störanfällige freie Kabelverbindung darstellt, wodurch die Benutzerfreundlichkeit verbessert wird.

Mit weiterem Vorteil weist das Verbindungselement mindestens zwei elektrische Leitungen auf. Damit ist die Möglichkeit geschaffen, ausgehend vom Atemluftbefeuchter nicht nur die Schlauchheizung des Exspirationsschlauches zu steuern, sondern auch Mess- oder Datenleitungen im Exspirationsschlauch anzubringen, beispielsweise für einen oder mehrere Temperatursensoren. Zusätzliche, störende Kabel werden dadurch vermieden.

In bevorzugter Weise weist der Exspirationsschlauch zur Verbindung mit dem Beatmungsgerät ein erstes Anschlussstück auf und der zweite Inspirationsschlauch weist zur Verbindung mit dem Beatmungsgerät ein zweites Anschlussstück auf, wobei das Verbindungselement mit dem ersten und zweiten Anschlussstück verbunden ist. Da die Anschlussstücke an das Beatmungsgerät normalerweise feste, extrudierte Kunststoffelemente sind, wird dadurch eine stabile, integrale Struktur geschaffen, die gegen Störungen und Funktionsfehler weniger anfällig ist. Dabei kann das Verbindungselement zweckmäßig zwei Steckelemente aufweisen, die in dafür vorgesehene Hülsenelemente auf dem ersten bzw. zweiten Anschlussstück einsteckbar sind. Die Verbindung zwischen den Steckelementen und dem ersten bzw. zweiten Anschlusselement ist bevorzugter Weise nicht lösbar, um für das Bedienpersonal nicht die Möglichkeit zu bieten, die elektrischen Verbindungen zu manipulieren.

Bevorzugt ist das Verbindungsstück U-förmig ausgebildet. Dies entspricht einer ergonomischen Struktur und im Wesentlichen dem Leitungsweg der Heiz-, Daten- oder Messleitungen auf dem Exspirationsschlauch und dem zweiten Inspirationsschlauch.

Mit weiterem Vorteil ist Heizdraht der Schlauchheizung zumindest abschnittsweise als Heizwendel ausgebildet. Die Heizwendel ist dabei beispielsweise in die Außenwand der Beatmungsschläuche integriert und sorgt damit für eine effektive Heizwirkung.

Besonders bevorzugt weist der erste Inspirationsschlauch und/oder zweite Inspirationsschlauch mindestens eine elektrische Mess- oder Datenleitung auf. Damit ist es beispielsweise möglich, Daten vom Beatmungsgerät über die elektrische Datenleitung an den Atemluftbefeuchter und weiter zum Patienten hin zu übertragen, so dass die Informationen über die Anzeige des Atemluftbefeuchters angezeigt werden können. Auch Daten, die am Y-Stück nahe am oder vom Patienten aufgenommen wurden, können weiter an den Atemluftbefeuchter oder an das Beatmungsgerät übertragen werden. Da die elektrischen Mess- oder Datenleitungen integral mit dem pneumatischen Schlauchelement ausgebildet sind, beispielsweise in die Schlauchhülle integriert, gibt es keine freien elektrischen Kabel, die störend wirken könnten und beschädigt werden können.

In gleicher Weise ist es vorteilhaft, dass der Exspirationsschlauch mindestens eine elektrische Mess- oder Datenleitung aufweist, die über den zweiten Inspirationsschlauch und das Verbindungselement geführt ist. Damit können Messsignale von Sensoren oder andere Daten, die im Exspirationsschlauch erfasst werden, über das Verbindungselement hin zum Atemluftbefeuchter geführt werden, um dort ausgewertet und zur Anzeige gebracht zu werden.

Ebenfalls ist es denkbar und vorteilhaft, dass die elektrische Mess- oder Datenleitung des ersten Inspirationsschlauches einen Temperatursensor aufweist. Der Temperatursensor sitzt üblicher Weise sehr nahe am Y-Stück, um die dortigen Temperaturverhältnisse nahe am Patienten zu messen. In diesem Zusammenhang ist es denkbar, dass die Temperaturmessleitung oder eine andere Leitung über ein Anschlussstück in das Y-Stück geführt ist, um für Übertragungen von Mess- oder Datensignalen aus Richtung des Patienten zur Verfügung zu stehen.

Vorteilhafter Weise ist der elektrische Widerstand der elektrischen Leitungen im zweiten Inspirationsschlauch, im Exspirationsschlauch und im Verbindungselement unterschiedlich. Die Leitungen im zweiten Inspirationsschlauch sowie im Verbindungselement können beispielsweise als elektrische Zuführleitungen für die Schlauchheizung des Exspirationsschlauches ausgeführt sein, und zwar derart, dass zum Beispiel die Heizleistung im Exspirationsschlauch und der Stromverbrauch optimiert sind. Dies erhöht auch die Zuverlässigkeit der Messungen bzw. der Schlauchheizung.

Besonders bevorzugt ist es, wenn das Beatmungsschlauchsystem als medizinischer Einmal-/Wegwerfartikel ausgebildet ist. Alternativ kann das Beatmungsschlauchsystem auch medizinisch wieder verwendbar ausgebildet sein. Um eine Kontamination zu vermeiden und damit die Infektionsgefahr für Patienten zu verringern, sind die meisten medizinischen Verbrauchsartikel als Einmal-/Wegwerfartikel ausgebildet. Dazu gehört auch das Beatmungsschlauchsystem der vorliegenden Erfindung, das möglichst nur an einem Patienten und nur für eine bestimmte Dauer verwendet werden soll. Es ist jedoch auch möglich, aus Kosten- oder anderen Gründen das Beatmungsschlauchsystem wieder verwendbar auszubilden, so dass das Beatmungsschlauchsystem in maschinell gereinigt und/oder autoklaviert werden kann, um danach wieder verwendet werden zu können.

Weiterhin erfindungsgemäß ist ein Beatmungssystem, das ein Beatmungsgerät, einen Atemluftbefeuchter und ein wie oben beschriebenes Beatmungsschlauchsystem umfasst, wobei der Atemluftbefeuchter eine Steuereinrichtung umfasst, die für die Stromversorgung und die Steuerung der Schlauchheizung für den Exspirationsschlauch und den ersten Inspirationsschlauch dient. Wie bereits erwähnt wird die Feuchtigkeit und die Temperatur des Atemgases üblicher Weise am Atemluftbefeuchter eingestellt. Dies bringt dem Benutzer den Vorteil, dass für die Zurverfügungstellung des Atemgases ein beliebiges geeignetes Beatmungsgerät verwendet werden kann, und die Funktion der Atemluftbefeuchtung und derart seine Steuerung unabhängig vom Beatmungsgerät in dem Atemluftbefeuchter erfolgen kann.

Mit anderen Worten heißt dies, dass das erfindungsgemäße Beatmungsschlauchsystem auch mit allen vorhandenen Beatmungsgeräten kombiniert werden kann, die über die üblichen genormten Schlauchanschlüsse für Inspirations- und Exspirationsschlauch verfügen.

Die Erfindung soll nachfolgend anhand von Ausführungsbeispielen unter Bezugnahme auf die beigefügten Figuren erläutert werden, in denen:
- Fig. 1: eine schematische Darstellung einer bevorzugten Ausführungsform des Beatmungsschlauchsystems der vorliegenden Erfindung zeigt; und
- Fig. 2: eine perspektivische Ansicht einer weiteren bevorzugten Ausführungsform des Beatmungsschlauchsystems gemäß der vorliegenden Erfindung zeigt.

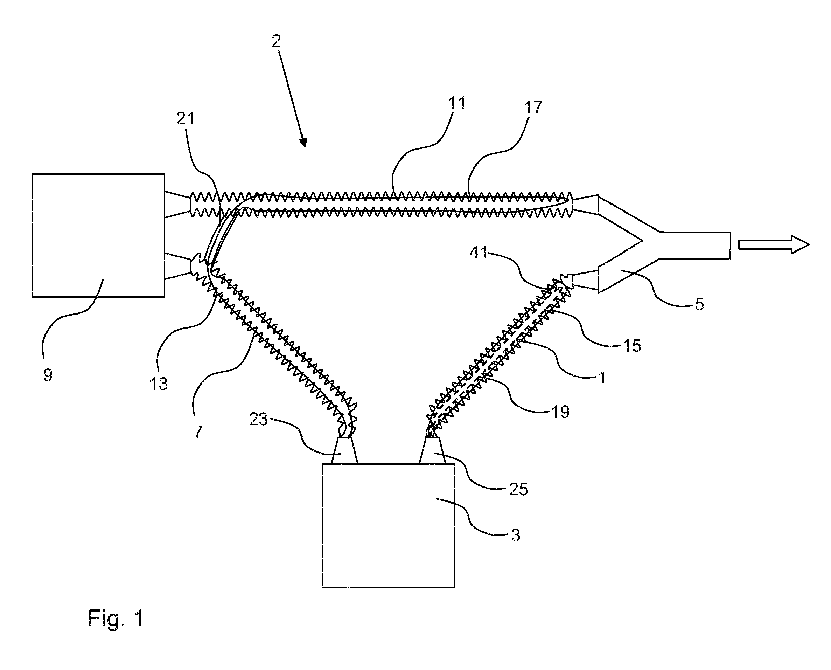

Fig. 1 zeigt in schematischer Darstellung eine bevorzugte Ausführungsform eines erfindungsgemäßen Beatmungsschlauchsystems. Ein erster Inspirationsschlauch 1 ist zwischen einem Atemluftbefeuchter 3 und einem Y-Stück 5 angeordnet. Das einfach ausgebildete Ende des Y-Stücks 5 zeigt mit dem dargestellten Pfeil in Richtung des zu beatmenden Patienten. Ein zweiter Inspirationsschlauch 7 verbindet den Atemluftbefeuchter 3 mit dem Beatmungsgerät 9. Schließlich ist ein Exspirationsschlauch 11 zwischen dem Beatmungsgerät 9 und dem verbleibenden Ende des Y-Stücks 5 angeordnet.

Trockenes Atemgas wird beispielsweise über ein (nicht dargestelltes) Gebläse im Beatmungsgerät 9 erzeugt und verlässt dieses über den zweiten Inspirationsschlauch 7 in Richtung des Atemluftbefeuchters 3. Dort wird das Atemgas in bekannter Art und Weise in einen (in Fig. 1 nicht dargestellten) Flüssigkeitsbehälter 31 geleitet, wo es durch die aufgeheizte Flüssigkeit erwärmt und befeuchtet wird. Über den ersten Inspirationsschlauch 1 verlässt das erwärmte und befeuchtete Atemgas den Atemluftbefeuchter und wird dem Patienten über das Y-Stück 5 zugeführt.

Entsprechend einem über das Beatmungsgerät 9 gesteuerten Atemzyklus verlässt die verbrauchte Atemluft den Patienten wieder und tritt am Y-Stück 5 in den Exspirationsschlauch 11 ein und wird wieder dem Beatmungsgerät 9 zugeführt.

In die Wand des ersten Inspirationsschlauches 1 ist ein Heizdraht 15 integriert, der beispielsweise spiralförmig als Heizwendel ausgebildet ist. Die Stromversorgung und Steuerung des Heizdrahtes 15 erfolgt ausgehend von der (nicht dargestellten) Stromversorgungs- und Steuereinrichtung im Atemluftbefeuchter 3 durch elektrische Anschlüsse in dem ersten Anschlussstück 25, das den ersten Inspirationsschlauch 1 mit dem Atemluftbefeuchter 3, genauer mit dem (in Fig. 1 nicht dargestellten) Flüssigkeitsbehälter 31 verbindet. Weiterhin in den ersten Inspirationsschlauch 1 integriert ist eine elektrische Messleitung 19, die das Signal von einem am Y-Stück 5 nahen Ende angeordneten Temperatursensor 41 zur (nicht dargestellten) Steuereinrichtung des Atemluftbefeuchters 3 überträgt. Der Ort des Temperatursensors 41 ist so gewählt, dass er möglichst nahe am Patienten liegt und doch noch Teil des möglichst einfach austauschbaren Beatmungsschlauchsystems ist. Auch die elektrische Messleitung 19 wird über Kontaktelemente vom ersten Anschlussstück 25 auf entsprechend dazu passende Kontakte am Atemluftbefeuchter 3 bzw. dessen Flüssigkeitsbehälter übertragen.

Auch der Exspirationsschlauch 11 weist eine Schlauchheizung in Form des Heizdrahtes 17 auf, der ebenfalls als Heizwendel spiralförmig ausgebildet ist. Der Grund für die Beheizung des Exspirationsschlauches 11 ist, dass vermieden werden soll, dass zurückströmendes Atemgas im Exspirationsschlauch 11 kondensiert und beispielsweise als verunreinigte Flüssigkeit über das Y-Stück 5 wieder zurück zum Patienten fließt. Die Schlauchheizung des Exspirationsschlauches 11 kann durchgängig oder abschnittsweise ausgebildet sein. Mit Strom versorgt wird der Heizdraht 17 von der Stromversorgungs- und Steuereinrichtung des Atemluftbefeuchters 3 über eine Stromversorgungsleitung 13, die ausgehend vom Atemluftbefeuchter 3 über ein zweites Anschlussstück 23 und den zweiten Inspirationsschlauch 7 sowie das Verbindungselement 21 bis zum Exspirationsschlauch 11 geführt ist und dort an den Heizdraht 17 angeschlossen ist. Durch die Führung über den zweiten Inspirationsschlauch 7 sowie das Verbindungselement 21 wird verhindert, dass die Stromversorgungsleitung 13 für den Exspirationsschlauch 11 frei als loses Kabel verläuft. Die Stromversorgungsleitung 13 bzw. der Heizdraht 17 kann so zusammen mit dem gesamten Beatmungsschlauchsystem zusammen als Einwegmaterial auf sehr einfache Weise ausgetauscht werden, da sie bzw. er nicht mit anderen Geräten wie z.B. dem Beatmungsgerät verbunden werden müssen.

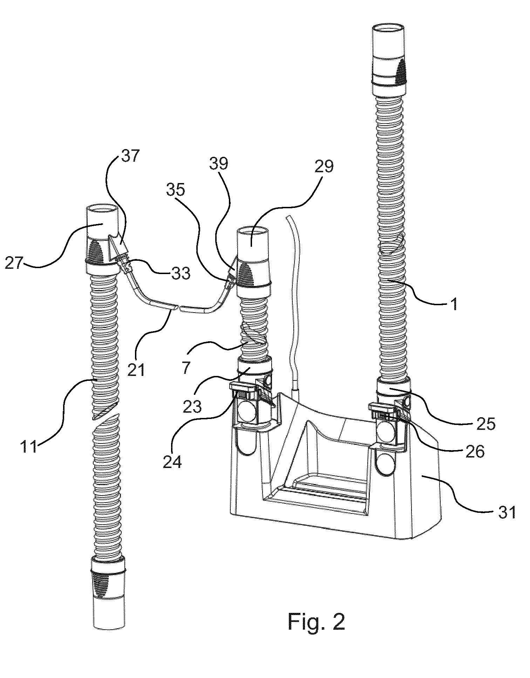

Das Verbindungselement 21 ist in Fig. 1 nahe an den Anschlussstücken zwischen Beatmungsgerät 9 und dem zweiten Inspirationsschlauch 7 bzw. dem Exspirationsschlauch 11 angeordnet. Ausführlich wird das Verbindungselement 21 nun unter Bezugnahme auf Fig. 2 beschrieben.

Fig. 2 zeigt eine perspektivische Darstellung des erfindungsgemäßen Beatmungsschlauchssystems, wobei der erste und zweite Inspirationsschlauch 1 bzw. 7 über entsprechende Anschlussstücke 23, 25 mit dem Flüssigkeitsbehälter 31 des (in Fig. 2 nicht dargestellten) Atemluftbefeuchters 3 verbunden sind. In Fig. 2 ist lediglich die pneumatische Verbindung zwischen erstem und zweitem Inspirationsschlauch 1, 7 und dem Flüssigkeitsbehälter 31 über die Anschlussstücke 23, 25 dargestellt, da die elektrische Verbindung sämtlicher in Schläuchen verlaufender elektrischen Verbindungen über Kontaktelemente 24, 26 erfolgt, die in die Anschlussstücke 23, 25 integriert sind und den elektrischen Kontakt zu entsprechenden Gegenstück der Kontaktelemente an dem Gehäuse des Atemluftbefeuchters 3 herstellen.

Auch ist in Fig. 2 das Y-Stück 5 nicht dargestellt. Vielmehr betrifft die Darstellung in Fig. 2 die Elemente, die üblicher Weise als medizinische Einmal-/Wegwerfartikel das notwendige austauschbare Zubehör für die Funktion des Atemluftbefeuchters 3 zusammen mit dem Beatmungsschlauchsystem bilden.

Da die wesentlichen Elemente bereits unter Bezugnahme auf Fig. 1 beschrieben wurden, wird an dieser Stelle auf eine Wiederholung verzichtet. Ausführlich eingegangen wird jedoch auf die Unterschiede zur lediglich schematisch dargestellten Ausführungsform aus Fig. 1.

Das Material für die Schläuche, d. h. für den ersten Inspirationsschlauch 1, den zweiten Inspirationsschlauch 7 und den Exspirationsschlauch 11 ist aus einem geeigneten Kunststoffmaterial wie z. B. Polyethylen oder Polypropylen gebildet. Andere geeignete Materialien sind ebenfalls möglich. Die Schläuche werden in bekannter Technik extrudiert bzw. koextrudiert. Der Innendurchmesser der Schläuche beträgt üblicher Weise etwa 19 mm bei einem Beatmungssystem für Erwachsene, es können auch geringere Durchmesser wie 12 mm oder 15 mm verwendet werden, beispielsweise für Beatmungssysteme in Kinderoder Säuglingsintensivstationen. Die Anschlussstücke, die den Übergang zwischen den Schläuchen der entsprechenden Geräte bzw. dem Y-Stück 5 bilden, sind ebenfalls aus Kunststoffmaterial extrudiert. Da im medizinischen Bereich an die Materialien hohe Anforderungen gestellt werden, müssen die Materialen der ISO-Norm 5367-2000 genügen. Wie bereits erwähnt ist das erfindungsgemäße Beatmungsschlauchsystem entweder als medizinischer Einmal-/Wegwerfartikel ausgebildet oder alternativ als wieder verwendbarer medizinischer Artikel, der mittels Waschen und Autoklavieren wieder in einen neu verwendbaren Zustand versetzt werden kann. Alle Bestandteile des Beatmungsschlauchsystems müssen auch derart ausgebildet sein, dass sie beispielsweise keine Schadstoffe enthalten und einem kalten Desinfektionsmittel wie z.B. CIDEX, Sekusept, Korsolex usw. widerstehen.

Das Verbindungselement 21 ist in der Fig. 2 dargestellten Ausführungsform eine nahe am Beatmungsgerät 9, aber doch getrennt davon angeordnete, flexible Brücke, wobei über diese Brücke der zweite Inspirationsschlauch elektrisch mit dem Exspirationsschlauch verbunden ist. Die Länge der flexiblen Brücke beträgt etwa zwischen 5 cm und 50 cm, bevorzugt etwa 10 cm. Das Anschlussstück 29 des zweiten Inspirationsschlauchs 7 weist neben der pneumatischen Öffnung ein Hülsenelement 39 auf, in das ein entsprechendes Steckelement 35 des Verbindungselements 21 unlösbar eingesteckt ist. In gleicher Weise weist das Anschlussstück 27 des Exspirationsschlauches 11 neben der pneumatischen Öffnung ein Hülsenelement 37 auf, in die das Steckelement 33 des Verbindungselements 21 unlösbar eingesteckt ist. Der Begriff "unlösbar" ist in diesem Zusammenhang so zu verstehen, dass es für Bedienpersonal nicht ohne Weiteres möglich ist, die elektrischen Verbindungen zwischen Verbindungselement 21 und den Anschlussstücken 27, 29 zu lösen.

Das Verbindungselement 21 kann mehrere elektrische Leitungen aufweisen, und nicht nur zwei, wie im Ausführungsbeispiel aus Fig. 1 dargestellt, wo lediglich die Stromversorgungsleitungen für den Heizdraht des Exspirationsschlauches 11 enthalten sind. Beispielsweise können weitere Heizfunktionen für Sensoren, Filter und Ähnliches oder andere Sensoren elektrisch angebunden sein. Es ist ebenfalls möglich, eine Signalübertragung zwischen Exspirationsschlauch 11 und dem Atemluftbefeuchter 3 über das Verbindungselement 21 zu führen. Das Verbindungselement 21 zwischen ersten Inspirationsschlauch 7 und Exspirationsschlauch 11 kann so ausgebildet sein, dass eine laterale Verschiebung zwischen beiden Schlauchenden leicht möglich ist, dass jedoch eine gegenseitige Verdrehung erschwert ist.

Mit dem Gegenstand der vorliegenden Erfindung wurde ein Beatmungsschlauchsystem bereit gestellt, das die Anzahl der Schläuche und elektrischen Leitungen sowie deren Anschlüsse minimiert, flexible Anschlussmöglichkeiten bietet und eine Verbindung von pneumatischen und elektrischen Anschlüssen in einem Verbindungsvorgang ermöglicht, ohne dass sich eine Bedienperson oder der Patient in zusätzlichen elektrischen Leitungen der Heizdrähte verheddert und deren elektrische Verbindung kappt.

## Patentansprüche

1. Beatmungsschlauchsystem für Atemgas zum Beatmen von Patienten mit
einem ersten Inspirationsschlauch (1), der mit einem Atemluftbefeuchter (3) und mit einem Y-Stück (5) verbindbar ist,
einem zweiten Inspirationsschlauch (7), der mit einem Beatmungsgerät (9) und mit dem Atemluftbefeuchter (3) verbindbar ist, und
einem Exspirationsschlauch (11), der mit dem Beatmungsgerät (9) und mit dem Y-Stück (5) verbindbar ist,
wobei der erste Inspirationsschlauch (1) und der Exspirationsschlauch (11) wenigstens abschnittsweise jeweils eine Schlauchheizung mit mindestens einem Heizdraht (15, 17) aufweisen, die von einer Stromversorgungs- und Steuereinrichtung innerhalb des Atemluftbefeuchters (3) steuerbar ist,
**dadurch gekennzeichnet, dass**
der zweite Inspirationsschlauch (7) und der erste Inspirationsschlauch (1) jeweils ein Anschlussstück (23, 25) zur pneumatischen und elektrischen Verbindung mit dem Atemluftbefeuchter (3) aufweisen,
wobei eine Stromversorgungsleitung (13) der Schlauchheizung des Exspirationsschlauchs (11) ausgehend von dem Anschlussstück (23) des zweiten Inspirationsschlauchs (7) über den zweiten Inspirationsschlauch (7) und ein von dem Beatmungsgerät (9) separates Verbindungselement (21) geführt ist.

2. Beatmungsschlauchsystem nach Anspruch 1, **dadurch gekennzeichnet, dass** die Anschlussstücke (23, 25) mit dem Atemluftbefeuchter (3) derart verbindbar sind, dass die pneumatische Verbindung der Atemgaswege zusammen mit der elektrischen Verbindung bewirkt wird.

3. Beatmungsschlauchsystem nach einem der vorhergehenden Ansprüche, **dadurch gekennzeichnet, dass** das Verbindungselement (21) als flexible Brücke ausgebildet ist, die den Exspirationsschlauch (11) und den zweiten Inspirationsschlauch (7) im Bereich ihrer beatmungsgeräteseitigen Enden verbindet.

4. Beatmungsschlauchsystem nach einem der vorhergehenden Ansprüche, **dadurch gekennzeichnet, dass** das Verbindungselement (21) mindestens zwei elektrische Leitungen aufweist.

5. Beatmungsschlauchsystem nach einem der vorhergehenden Ansprüche, **dadurch gekennzeichnet, dass** der Exspirationsschlauch (11) zur Verbindung mit dem Beatmungsgerät (9) ein erstes Anschlussstück (27) aufweist und der zweite Inspirationsschlauch (7) zur Verbindung mit dem Beatmungsgerät (9) ein zweites Anschlussstück (29) aufweist, wobei das Verbindungselement (21) mit dem ersten und zweiten Anschlussstück (27, 29) verbunden ist.

6. Beatmungsschlauchsystem nach Anspruch 5, **dadurch gekennzeichnet, dass** das Verbindungselement (21) zwei Steckelemente (33, 35) aufweist, die in dafür vorgesehene Hülsenelemente (37, 39) auf dem ersten bzw. zweiten Anschlussstück (27, 29) einsteckbar sind.

7. Beatmungsschlauchsystem nach Anspruch 6, **dadurch gekennzeichnet, dass** die Verbindung zwischen den Steckelementen (33, 35) und dem ersten bzw. zweiten Anschlussstück (27, 29) nicht lösbar ist.

8. Beatmungsschlauchsystem nach einem der vorhergehenden Ansprüche, **dadurch gekennzeichnet, dass** das Verbindungselement (21) U-förmig ausgebildet ist.

9. Beatmungsschlauchsystem nach einem der vorhergehenden Ansprüche, **dadurch gekennzeichnet, dass** der Heizdraht (15, 17) der Schlauchheizung zumindest abschnittsweise als Heizwendel ausgebildet ist.

10. Beatmungsschlauchsystem nach einem der vorhergehenden Ansprüche, **dadurch gekennzeichnet, dass** der erste Inspirationsschlauch (1) und/oder zweite Inspirationsschlauch (7) mindestens eine elektrische Mess- oder Datenleitung aufweist.

11. Beatmungsschlauchsystem nach Anspruch 10, **dadurch gekennzeichnet, dass** der Exspirationsschlauch (11) mindestens eine elektrische Mess- oder Datenleitung aufweist, die über den zweiten Inspirationsschlauch (7) und das Verbindungselement geführt (21) ist.

12. Beatmungsschlauchsystem nach Anspruch 10, **dadurch gekennzeichnet, dass** die elektrische Mess- oder Datenleitung mit dem Beatmungsgerät (9) verbindbar ist.

13. Beatmungsschlauchsystem nach einem der vorhergehenden Ansprüche, **dadurch gekennzeichnet, dass** die elektrische Mess- oder Datenleitung des ersten Inspirationsschlauchs (1) einen Temperatursensor (41) aufweist.

14. Beatmungsschlauchsystem nach einem der vorhergehenden Ansprüche, **dadurch gekennzeichnet, dass** der elektrische Widerstand der elektrischen Leitungen im zweiten Inspirationsschlauch (7), im Exspirationsschlauch (11) und im Verbindungselement (21) unterschiedlich ist.

15. Beatmungsschlauchsystem nach einem der vorhergehenden Ansprüche, **dadurch gekennzeichnet, dass** es als medizinischer Einmal-/Wegwerfartikel oder als medizinisch wieder verwendbar ausgebildet ist.

16. Beatmungssystem (2) umfassend ein Beatmungsgerät (9), einen Atemluftbefeuchter (3) und ein Beatmungsschlauchsystem nach einem der vorhergehenden Ansprüche, **dadurch gekennzeichnet, dass** der Atemluftbefeuchter (3) eine Steuereinrichtung umfasst, die zur Stromversorgung und zur Steuerung der Schlauchheizung für den Exspirationsschlauch (11) und den ersten Inspirationsschlauch (1) dient.

## Claims

1. A ventilation tube system for breathing gas for ventilating patients having
a first inhalation tube (1) connectable to a respiratory humidifier (3) and to a Y-piece (5);
a second inhalation tube (7) connectable to a respirator (9) and to the respiratory humidifier (3); and
an exhalation tube (11) connectable to the respirator (9) and to the Y-piece (5);
wherein at least certain portions of the first inhalation tube (1) and the exhalation tube (11) each comprise tube heaters having at least one heating wire (15, 17),
wherein each of the tube heaters is controllable by a power supply and control unit inside the respiratory humidifier (3),
**characterized in that**
the second inhalation tube (7) and the first inhalation tube (1) each comprise a connecting piece (23, 25) for the pneumatic and electrical connection to the respiratory humidifier (3),
wherein a power supply line (13) of the tube heater of the exhalation tube (11) starts from the connecting piece (23) of the second inhalation tube (7) and proceeds from there via the second inhalation tube (7) and a connecting element (21) which is separate from the respirator (9).

2. The ventilation tube system according to claim 1, **characterized in that** the connecting pieces (23, 25) are connectable to the respiratory humidifier (3) in such a way that the pneumatic connection of the breathing gas pathway is effected together with the electrical connection.

3. The ventilation tube system according to one of the preceding claims, **characterized in that** the connecting element (21) is designed as a flexible bridge connecting the exhalation tube (11) and the second inhalation tube (7) in the area of their respirator-facing ends.

4. The ventilation tube system according to one of the preceding claims, **characterized in that** the connecting element (21) comprises at least two electrical lines.

5. The ventilation tube system according to one of the preceding claims, **characterized in that** the exhalation tube (11) comprises a first connecting piece (27) for connecting to the respirator (9), and the second inhalation tube (7) comprises a second connecting piece (29) for connecting to the respirator (9), wherein the connecting element (21) is connected to the first and second connecting pieces (27, 29).

6. The ventilation tube system according to claim 5, **characterized in that** the connecting element (21) comprises two plug elements (33, 35), which are insertable into socket elements (37, 39) provided for the purpose on the first and second connecting pieces (27, 29).

7. The ventilation tube system according to claim 6, **characterized in that** the connections between the plug elements (33, 35) and the first and second connecting pieces (27, 29) are not detachable.

8. The ventilation tube system according to one of the preceding claims, **characterized in that** the connecting element (21) is formed in the shape of a "U".

9. The ventilation tube system according to one of the preceding claims, **characterized in that** at least certain portions of the heating wire (15, 17) of the tube heater are formed as heating coils.

10. The ventilation tube system according to one of the preceding claims, **characterized in that** the first inhalation tube (1) and/or the second inhalation tube (7) comprises at least one electrical measurement or data line.

11. The ventilation tube system according to claim 10, **characterized in that** the exhalation tube (11) comprises at least one electrical measurement or data line, which continues along the second inhalation tube (7) and the connecting element (21).

12. The ventilation tube system according to claim 10, **characterized in that** the electrical measurement or data line is connectable to the respirator (9).

13. The ventilation tube system according to one of the preceding claims, **characterized in that** the electrical measurement or data line of the first inhalation tube (1) comprises a temperature sensor (41).

14. The ventilation tube system according to one of the preceding claims, **characterized in that** the electrical resistance of the electrical lines in the second inhalation tube (7), in the exhalation tube (11), and in the connecting element (21) is different.

15. The ventilation tube system according to one of the preceding claims, **characterized in that** it is designed as a medical-grade single-use / disposable article or as a medically reusable article.

16. A ventilation system (2) comprising a respirator (9), a respiratory humidifier (3), and a ventilation tube system according to one of the preceding claims, **characterized in that** the respiratory humidifier (3) comprises a control unit serving to supply power to and to control the tube heaters for the exhalation tube (11) and the first inhalation tube (1).

## Revendications

1. Système de tubes de ventilation pour gaz respiratoire pour ventiler des patients, comprenant
un premier tube d'inspiration (1) qui peut être relié à un humidificateur d'air respiratoire (3) et à une pièce en Y (5),
un deuxième tube d'inspiration (7) qui peut être relié à un appareil de ventilation (9) et à l'humidificateur d'air respiratoire (3), et
un tube d'expiration (11) qui peut être relié à l'appareil de ventilation (9) et à la pièce en Y (5),
dans lequel le premier tube d'inspiration (1) et le tube d'expiration (11) présentent au moins par endroits respectivement un chauffage de tube comprenant au moins un fil chauffant (15, 17) qui peut être commandé par un dispositif d'alimentation électrique et de commande à l'intérieur de l'humidificateur d'air respiratoire (3),
**caractérisé en ce que**
le deuxième tube d'inspiration (7) et le premier tube d'inspiration (1) présentent respectivement une pièce de raccordement (23, 25) pour la liaison pneumatique et électrique avec l'humidificateur d'air respiratoire (3),
dans lequel une ligne d'alimentation électrique (13) du chauffage de tube du tube d'expiration (11) à partir de la pièce de raccordement (23) du deuxième tube d'inspiration (7) passe par le deuxième tube d'inspiration (7) et un élément de liaison (21) séparé de l'appareil de ventilation (9).

2. Système de tubes de ventilation selon la revendication 1, **caractérisé en ce que** les pièces de raccordement (23, 25) peuvent être reliées à l'humidificateur d'air respiratoire (3) de telle sorte que la liaison pneumatique des voies de gaz respiratoire soit réalisée conjointement avec la liaison électrique.

3. Système de tubes de ventilation selon l'une des revendications précédentes, **caractérisé en ce que** l'élément de liaison (21) est conçu comme un pont flexible qui relie le tube d'expiration (11) et le deuxième tube d'inspiration (7) dans la zone de leurs extrémités du côté de l'appareil de ventilation.

4. Système de tubes de ventilation selon l'une des revendications précédentes, **caractérisé en ce que** l'élément de liaison (21) présente au moins deux lignes électriques.

5. Système de tubes de ventilation selon l'une des revendications précédentes, **caractérisé en ce que** le tube d'expiration (11) présente, pour la liaison avec l'appareil de ventilation (9), une première pièce de raccordement (27) et le deuxième tube d'inspiration (7) présente, pour la liaison avec l'appareil de ventilation (9), une deuxième pièce de raccordement (29), dans lequel l'élément de liaison (21) est lié à la première et la deuxième pièce de raccordement (27, 29) .

6. Système de tubes de ventilation selon la revendication 5, **caractérisé en ce que** l'élément de liaison (21) présente deux éléments enfichables (33, 35) qui peuvent être enfichés dans des éléments de manchon (37, 39) prévus à cet effet sur la première ou la deuxième pièce de raccordement (27, 29).

7. Système de tubes de ventilation selon la revendication 6, **caractérisé en ce que** la liaison entre les éléments enfichables (33, 35) et la première ou la deuxième pièce de raccordement (27, 29) n'est pas détachable.

8. Système de tubes de ventilation selon l'une des revendications précédentes, **caractérisé en ce que** l'élément de liaison (21) est construit en forme de U.

9. Système de tubes de ventilation selon l'une des revendications précédentes, **caractérisé en ce que** le fil chauffant (15, 17) du chauffage de tube est formé au moins par endroits comme une spirale chauffante.

10. Système de tubes de ventilation selon l'une des revendications précédentes, **caractérisé en ce que** le premier tube d'inspiration (1) et/ou le deuxième tube d'inspiration (7) présente(nt) au moins une ligne électrique de mesure ou de données.

11. Système de tubes de ventilation selon la revendication 10, **caractérisé en ce que** le tube d'expiration (11) présente au moins une ligne électrique de mesure ou de données qui passe par le deuxième tube d'inspiration (7) et l'élément de liaison (21) .

12. Système de tubes de ventilation selon la revendication 10, **caractérisé en ce que** la ligne électrique de mesure ou de données peut être reliée à l'appareil de ventilation (9).

13. Système de tubes de ventilation selon l'une des revendications précédentes, **caractérisé en ce que** la ligne électrique de mesure ou de données du premier tube d'inspiration (1) présente un capteur de température (41).

14. Système de tubes de ventilation selon l'une des revendications précédentes, **caractérisé en ce que** la résistance électrique des lignes électriques est différente dans le deuxième tube d'inspiration (7), dans le tube d'expiration (11) et dans l'élément de liaison (21).

15. Système de tubes de ventilation selon l'une des revendications précédentes, **caractérisé en ce qu'**il est conçu comme un article médical à usage unique/jetable ou comme un article médicalement réutilisable.

16. Système de tubes de ventilation (2) comprenant un appareil de ventilation (9), un humidificateur d'air respiratoire (3) et un système de tubes de ventilation selon l'une des revendications précédentes, **caractérisé en ce que** l'humidificateur d'air respiratoire (3) comprend un dispositif de commande qui sert à l'alimentation électrique et à la commande du chauffage de tube pour le tube d'expiration (11) et le premier tube d'inspiration (1).
